# EUROPÄISCHE PATENTSCHRIFT

(11) **EP 3 570 812 B1**
(45) Veröffentlichungstag und Bekanntmachung des Hinweises auf die Patenterteilung: **26.07.2023**
(21) Anmeldenummer: 17816681.5
(22) Anmeldetag: 11.12.2017
(51) Int. Cl.: A61K 8/34, A61Q 1/02, A61K 8/02

(54) **KOSMETISCHE ZUBEREITUNGEN MIT EINEM GEHALT AN SPHÄRISCHEN SILICAPARTIKELN, WELCHE MIT TITANDIOXID UND EISENOXIDEN BESCHICHTET SIND UND MITTELKETTIGEN ALIPHATISCHEN DIOLEN**
COSMETIC PREPARATIONS CONTAINING SPHEREIC SILICONE MATERIALS COATED WITH TITANIUM DIOXIDE AND IRON OXIDES AND MEDIUM-CHAINED ALIPHATIC DIOLES
PRÉPARATIONS COSMÉTIQUES CONTENANT UNE TENEUR EN MATÉRIAUX DE SILICONE SPHÉRIQUE REVÊTUS DE DIOXYDE DE TITANE ET D'OXYDES DE FER ET DE DIOLES ALIPHATIQUES À CHAÎNE MOYENNE

(30) Priorität: 20.01.2017 DE 102017200921
(43) Veröffentlichungstag der Anmeldung: 27.11.2019
(73) Patentinhaber: Beiersdorf AG, 20253 Hamburg (DE)
(72) Erfinder: CHANTY, Delphine, 20253 Hamburg (DE); RASCHKE, Thomas, 25421 Pinneberg (DE); NISSEN, Bente, 20144 Hamburg (DE)
(74) Vertreter: Beiersdorf AG
(86) Internationale Anmeldenummer: PCT/EP2017/082156
(87) Internationale Veröffentlichungsnummer: WO 2018/133994

(56) Entgegenhaltungen:
- EP-A1- 1 946 742
- WO-A1-00/15720

## Beschreibung

Die Erfindung betrifft kosmetische oder dermatologische Zubereitungen mit einem Gehalt an sphärischen Silicapartikeln, welche mit Titandioxid und Eisenoxiden beschichtet sind und mittelkettigen aliphatischen Diolen, wie in Anspruch 1 definiert.

Viele Körperpflegeprodukte, die derzeit für den Verbraucher erhältlich sind, sind in erster Linie darauf gerichtet, die Gesundheit oder das äußere Erscheinungsbild der Haut oder des Haares zu verbessern. Von den Hautpflegeprodukten sind viele darauf gerichtet, die Faltenbildung der Haut und andere histologische Veränderungen, die in der Regel mit der Hautalterung oder mit einer Schädigung der Haut durch Umwelteinflüsse in Zusammenhang stehen, zu verzögern, zu minimieren oder sogar zu eliminieren.

Es wurden in der Technik zahlreiche Verbindungen als geeignet für die Regulierung des Hautzustandes, einschließlich der Regulierung feiner Linien, Falten und anderer Formen von ungleichmäßiger oder rauer Oberflächenbeschaffenheit im Zusammenhang mit gealterter oder lichtgeschädigter Haut beschrieben.

EP 1 946 742 offenbart kosmetische Wasser-in-Silikonöl-Emulsion enthaltend 1 ,2-Decandiol und beschichtetes Silica wie Ronasphere LDP von Merck (Silica + Titanium Dioxide + iron Oxides).

Die Haut ist Belastungen durch zahlreiche äußere und innere Faktoren ausgesetzt. Extrinsische Faktoren umfassen Ultraviolettstrahlung (z. B. Sonnenbestrahlung), Umweltverschmutzung, Wind, Hitze, niedrige Feuchtigkeit, aggressive Tenside, Schleifmittel und dergleichen. Intrinsische Faktoren umfassen chronologisches Alter und andere biochemische Veränderungen von innerhalb der Haut. Ob nun äußerlich oder innerlich, diese Faktoren resultieren in sichtbaren Zeichen der Hautalterung und der Schädigung durch Umwelteinflüsse, wie der Entstehung von Falten und anderen Formen der Rauigkeit (einschließlich vergrößerter Poren, Schuppenbildung und Hautlinien) sowie anderen histologischen Veränderungen im Zusammenhang mit Hautalterung oder -schädigung. Hautfalten erinnern viele Menschen an das Verschwinden der Jugend.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff "Senile Xerosis" fallen können:
a) Trockenheit, Rauigkeit und Ausbildung von Trockenheitsfältchen,
b) Juckreiz
   und
c) verminderte Rückfettung durch Talgdrüsen (z. B. nach Waschen).

Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgenden Strukturschäden- und Funktionsstörungen in der Haut, die über Maß und Qualität der Schäden bei chronologischer Alterung hinausgehen:
d) sichtbare Gefäßerweiterungen (Teleangiektasien, Cuperosis),
e) Schlaffheit und Ausbildung von Falten,
f) lokale Hyper-, Hypo- und Fehlpigmentierungen (z. B. Altersflecken)
   und
g) vergrößerte Anfälligkeit gegenüber mechanischem Stress (z. B. Rissigkeit).

Die vorliegende Erfindung betrifft insbesondere Produkte zur Pflege der auf natürliche Weise gealterten Haut, sowie zur Behandlung der Folgeschäden der Lichtalterung, insbesondere der unter a) bis g) aufgeführten Phänomenen.

Hautunebenheiten, beispielsweise kleine Fältchen, sind besonders unerwünschte Begleiterscheinungen der Hautalterung. Eines der Ziele kosmetischer Mittel ist es, diese Erscheinung entweder zu beseitigen, zu überspielen und/oder zu minimeren. Ein Weg besteht darin, der Haut Pflegekomponenten oder Wirkstoffe zuzufügen, was aber ein langwieriges Unterfangen darstellen kann.

Produkte zur Pflege gealterter Haut enthalten z. B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft Hautreizungen. Retinoide sind daher nur in geringen Konzentrationen einsetzbar. Erwünscht ist dabei ein sofort nach der Anwendung des kosmetischen Mittels sichtbarer Effekt, wobei aber andererseits verhindert werden soll, dass eine "maskenhafte" Anmutung des betroffenen Hautareals bewirkt wird.

Es ist bekannt, dass durch die Verwendung sogenannter "Soft-Focus-Rohstoffe" kleinere Hautunebenheiten und Fältchen optisch kaschiert werden können. Solche Rohstoffe zeichnen sich durch eine geeignete Lichtbrechung und Lichtstreuung aus und mildern durch ihr diffuses Streuverhalten die Oberflächenerscheinungen der Haut.

Pigmente entstehen typischerweise in Form der Primärteilchen. Die Primärteilchen können über ihre Flächen zu Aggregaten zusammenwachsen. Von Agglomeraten spricht man, wenn Primärteilchen und/oder Aggregate über ihre Ecken/Kanten verbunden sind. Durch den Dispergierprozess (Dispergierung) beim Einarbeiten der Pigmente in ein Anwendungsmedium werden die Pigment-Agglomerate durch mechanische Scherung zerkleinert. Es entstehen kleinere Agglomerate, Aggregate und Primärteilchen. Diese werden, so vorhanden, durch ein Dispergiermedium benetzt. Dabei werden sie idealerweise statistisch über das Anwendungsmedium verteilt.

Es sind zahlreiche Pigmente mit "Soft-Focus-Effect" bekannt. Problematisch ist indessen, dass solche Pigmente, wie viele Festkörper, sich oft nur durch formulatorische Kniffe stabil in kosmetische oder dermatologische Zubereitungen einarbeiten lassen.

Eine weitere Aufgabe war es, kosmetische Zubereitungen zur Verfügung zu stellen, die einerseits breite Einsatzmöglichkeiten bieten, andererseits auch nach Anwendung ein möglichst natürlich anmutendes Erscheinungsbild der Haut gewährleisten und gleichzeitig die Anwesenheit von Hautunebenheiten, insbesondere kleiner Fältchen, zu vermindern oder sogar gänzlich zu kaschieren.

Gelöst wurde diese Aufgabe durch kosmetische oder dermatologische Zubereitungen mit einem Gehalt an sphärischen Silicapartikeln, welche mit Titandioxid und Eisenoxiden beschichtet sind und mittelkettigen aliphatischen Diolen, welche gewählt werden aus der Gruppe Octandiol, Ethylhexylglycerin und/oder Glycerylcaprylat.

Die erfindungsgemäß verwendeten mittelkettigen aliphatischen Diole verbessern die Einarbeitbarkeit der erfindungsgemäßen verwendeten beschichteten sphärischen Silicapartikel erheblich, insbesondere im Vergleich zu niederkettigen aliphatischen Diolen, beispielsweise Hexandiol, Propylenglycol und Dipropylenglycol.

Ethylhexylglycerin zeichnet sich durch folgende Struktur aus:

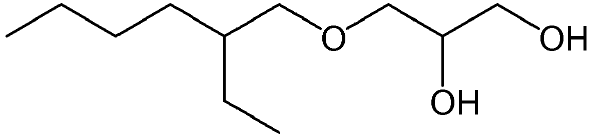

Besonders vorteilhafte sphärische Silicapartikel, welche mit Titandioxid und Eisenoxiden beschichtet sind, sind "Soft-Focus-Pigmente" und werden von sphärischen Silica-Partikeln abgeleitet, die mit Titandioxid und Eisenoxiden beschichtet sind. Solche Produkte sind beispielsweise unter der Bezeichnung "RonaFlair^{®} Flawless" von Merck erhältlich.

Vertreter dieser so genannten "Soft-Focus-Pigmente" oder Mikropigmente haben eine mittlere Korngröße von 4.0 - 8.0 µm und haben folgende typische Zusammensetzung: 74.0 - 85.0 % Siliciumdioxid (Silica), 15.0 - 24.0 % Titandioxid und ≤ 2.0% Eisenoxide. Aufgrund ihrer Zusammensetzung und Struktur zeigen Dispersionen dieser Mikropigmente eine leichte rosa Eigenfärbung.

Bevorzugt enthalten kosmetische oder dermatologische Zubereitungen gemäß der Erfindung 0,01 - 30 Gew.-%, bevorzugt 0,1 - 20 Gew.-%, besonders bevorzugt 0,2 - 10 Gew.-% an einem oder mehreren der erfindungsgemäßen mittelkettigen aliphatischen Diole, bezogen auf die Gesamtzusammensetzung der Zubereitungen, insbesondere ein oder mehrere Diole, gewählt aus der Gruppe Octandiol, Ethylhexylglycerin und Glycerylcaprylat. Bevorzugt enthalten kosmetische oder dermatologische Zubereitungen gemäß der Erfindung 0,1 - 20 Gew.-%, bevorzugt 0,5 - 15 Gew.-%, besonders bevorzugt 1 - 10 Gew.-% an einem oder mehreren der erfindungsgemäßen "Soft-Focus-Pigmente", bezogen auf die Gesamtzusammensetzung der Zubereitungen.

Emulsionen sind vorteilhafte Darreichungsformen im Sinne der vorliegenden Erfindung, z. B. in Form einer Crème, einer Lotion, einer kosmetischen Milch sind vorteilhaft und enthalten z. B. Fette, Öle, Wachse und/oder andere Fettkörper, sowie Wasser und einen oder mehrere Emulgatoren, wie sie üblicherweise für einen solchen Typ der Formulierung verwendet werden.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wässrige Lösungen
- Öle, wie Triglyceride der Caprin- oder der Caprylsäure; Siliconöle
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren; dreidimensional vernetzte Organopolysiloxane wie zum Beispiel Siloxanelastomere
- Alkohole, Diole oder Polyole niedriger C-Zahl, sowie deren Ether, vorzugsweise Ethanol, Isopropanol, Propylenglykol, Glycerin, Hexandiol, Octandiol, Ethylhexylglycerin, Glycerylcaprylat, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, Cetearylisononanoat, Isodecylneopentanoat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft.

Bei den erfindungsgemäßen Silikonelastomeren handelt es sich um vernetzte, gegebenenfalls substituierte Organopolysiloxane, die in einem geeigneten Trägermedium gequollen sind. Die Vernetzung kann mittels aller gängigen Verfahren und Vernetzern durchgeführt worden sein. Trägermedien können z. B. cyclische oder lineare Silikone sowie organische Lipide sein. Geeignete kommerziell erhältliche Silikonelastomergele sind z. B. KSG-15, KSG-16, KSG-18, KSG-210, KSG-310, KSG-320, KSG-330, KSG-340, KSG-41, KSG-42, KSG-43, KSG-44, KSG-710, KSG-810, KSG-820, KSG-830, KSG-840 von Shin Etsu, Velvesil DM, Velvesil 125, Velvesil Plus, oder andere Velvesil-Gel Typen oder Silsoft Silicon Gele von Momentive, oder EL-8040 ID, EL-8050 ID, EL-8051 IN, EL-8052, 9040, 9041, 9045, oder EL-9140 DM von Dow Corning.

Die wässrige Phase der erfindungsgemäßen Zubereitungen enthält gegebenenfalls vorteilhaft ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Celluloseether wie z. B. Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, Ultrez 10, ETD 2020, Acrylates/C10-30 Alkyl Acrylate Crosspolymer, Carbomer, Sodium Acrylates Copolymer jeweils einzeln oder in Kombination.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus (Metall)-Chelatoren, α-Hydroxysäuren (z. B. Citronensäure, Milchsäure), EDTA, ungesättigte Fettsäuren und deren Derivate (z. B. γ-Linoiensäure, Linolsäure, Ölsäure), Folsäure und deren Derivate, Ubichinon und deren Derivate (insbesondere Ubichinon Q10), Vitamin C und Derivate (z. B. Ascorbylpalmitat, Na-Ascorbylphosphat), Tocopherole und Derivate (z. B. Vitamin-E-acetat), Vitamin A und Derivate (Vitamin-A-palmitat), pflanzliche Polyphenole, Isoflavone, Genistein, Genistin, α-Glycosylrutin.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E (alpha-Tocopherol) und dessen Derivate sowie Vitamin A (Retinol) und dessen Derivate.

Die Menge der Antioxidantien (eine oder mehrere Verbindungen) in den Zubereitungen beträgt vorzugsweise 0,001 bis 10 Gew.-%, besonders bevorzugt 0,05 bis 5 Gew.-%, insbesondere 0,1 bis 1 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 1 Gew.-%, bezogen auf das Gesamtgewicht der Formulierung, zu wählen.

Sofern Vitamin A bzw. Vitamin-A-Derivate bzw. Carotine bzw. deren Derivate oder Analoga das oder die Antioxidantien darstellen, ist vorteilhaft, deren jeweilige Konzentrationen aus dem Bereich von 0,001 bis 1 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer, photochemischer und/oder radikalischer Beanspruchung schützen können.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit und dergleichen). Weiterhin vorteilhaft eignen sie sich gegen das Erscheinungsbild der trockenen bzw. rauhen Haut.

Zubereitungen im Sinne der vorliegenden Erfindung können vorzugsweise mindestens eine weitere UV-A-, UV-B- und/oder Breitbandfiltersubstanz enthalten. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Besonders vorteilhafte bei Raumtemperatur flüssige UV-Filtersubstanzen im Sinne der vorliegenden Erfindung sind 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure(2-ethylhexyl)ester (2-Ethylhexyl-4-methoxycinnamat, INCI: Octyl Methoxycinnamate).

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex^{®} 9020 verkauft wird.

Vorteilhafte weitere UV-Filtersubstanzen im Sinne der vorliegenden Erfindung sind sulfonierte, wasserlösliche UV-Filter, wie z. B.:
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethyl-ene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich.
- Vorteilhafte UV-Filtersubstanzen im Sinne der vorliegenden Erfindung sind ferner sogenannte Breitbandfilter, d. h. Filtersubstanzen, die sowohl UV-A- als auch UV-B-Strahlung absorbieren.
- Vorteilhafte Breitbandfilter oder UV-B-Filtersubstanzen sind beispielsweise Triazinderivate, wie z. B.:
   - 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb^{®} S bei der CIBA-Chemikalien GmbH erhältlich ist;
   - Diethylhexylbutylamidotriazon (INCI: Diethylhexylbutamidotriazone), welches unter der Handelsbezeichnung UVASORB HEB bei Sigma 3V erhältlich ist;
   - 4,4',4"-(1,3,5-Triazin-2,4,6-triyltriimino)-tris-benzoesäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung U-VINUL^{®} T 150 vertrieben wird.

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol), welches unter der Handelsbezeichnung Tinosorb^{®} M bei der CIBA-Chemikalien GmbH erhältlich ist.

Übliche Farbmittel, welche sich für die Verwendung als kosmetische oder dermatologische Zusammensetzungen im Sinne der vorliegenden Erfindung eignen, sind lipophilen Farbstoffen (z. B. Sudanrot, DB&C Red 17, DB&C Green 6, β-Carotin, Sudanbraun, DB&C Yellow 11, DB&C Violet 2, DB&C Orange 5 oder Annatto), hydrophilen Farbstoffen (z. B. Rote Beete-Saft oder Methylenblau), Pigmenten, Perlglanzpigmenten sowie Abmischungen aus solchen Farbmitteln.

Die Pigmente können weiß oder farbig, goniochromatisch oder nicht goniochromatisch, anorganisch und/oder organisch, beschichtet oder nicht beschichtet sein. Zu den anorganischen Pigmenten, die angegeben werden können, gehören Titandioxid, das optional oberflächenbehandelt ist, Zirconiumoxid (ZrO2), Zinkoxid (ZnO), Eisenoxid (z. B. Fe2O3), Siliciumoxid (SiO2), Mangans (z. B. MnO), Aluminiums (Al2O3), oder Ceroxid (z. B. Ce2O3), sowie Eisenoxid, Chromoxid, Manganviolett, Ultramarinblau, Chromhydrat und Eisenblau.

Die Perlglanzpigmente können unter weißen Perlglanzpigmenten, wie Glimmer, der mit Titan oder mit Bismutoxidchlorid überzogen ist, farbigen Perlglanzpigmenten, wie Titanglimmer mit Eisenoxiden, Titanglimmer mit vor allem Eisenblau oder Chromoxid, Titanglimmer mit einem organischen Pigment vom oben erwähnten Typ und Perlglanzpigmente auf der Basis von Bismutoxidchlorid ausgewählt werden.

Die Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Zur Herstellung der erfindungsgemäßen Experimente werden 15 Gew.-% Soft-Focus-Pigment" zusammen mit entmineralisiertem Wasser und 10 Gew.-% des jeweiligen Diols mit Magnetrührer für ca. 30 Minuten in 30-mL Gläsern gemischt. Danach wird der Rührprozess gestoppt und der Rührmagnet entnommen. Das Dispersionsverhalten des Pigments wird nach einer Stunde und nach 15 Stunden bewertet. Fotos werden zu beiden Zeitpunkten aufgenommen und die Redispergierung des Pigments durch Umdrehen der Gläser geprüft.

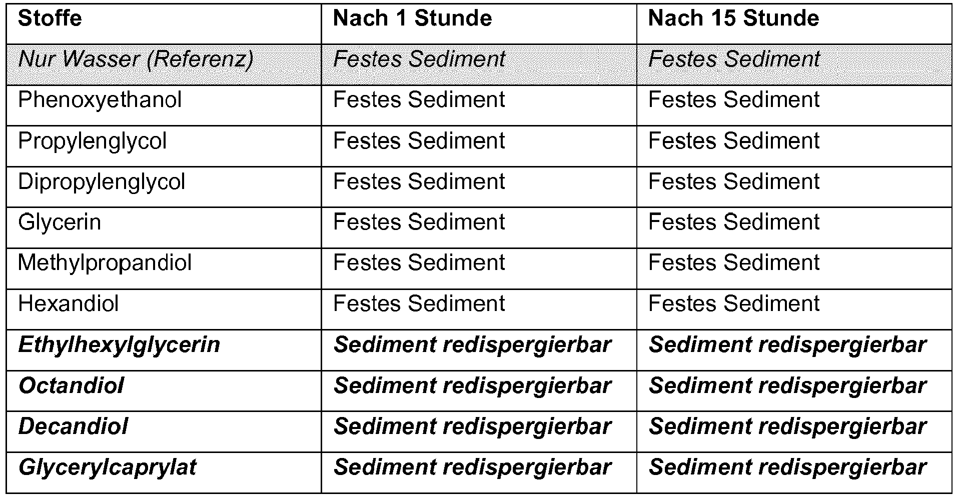

Die Dispersionen mit mittelkettigen, aliphatischen Diolen haben überraschenderweise eine deutlich bessere Redispergierbarkeit sowie auch eine bessere Stabilisierung und Pigmentverteilung des "Soft-Focus-Pigmentes" gezeigt als die Dispersionen mit niederkettigen aliphatischen Diolen.

Die folgenden Beispiele sollen die Erfindung näher erläutern. Wenn nicht anders angegeben, bedeuten die Angaben Gewichts-%.

| **Beispielrezeptur 1** | **Gew.-%** |
|---|---|
| Polyglyceryl-3 Methylglucosedistearat | 2,0 |
| Glycerylstearat | 1,0 |
| Isopropylpalmitat | 5 |
| Ethylhexyl Triazone | 1 |
| Aniso Triazin | 1 |
| Phenylbenzimidazole Sulfonsäure | 0,5 |
| Hydroxypropylmethylcellulose | 0,1 |
| Carbomer | 0,1 |
| Acrylates/C10-30 Alkyl Acrylate Crosspolymer | 0,2 |
| Octandiol | 0,5 |
| RonaFlair^{®} Flawless | 3 |
| Glycerin | 9 |
| Phenoxyethanol | 0,6 |
| EDTA | 1 |
| Kreatin | 0,2 |
| Ethanol | 3 |
| Tapiocastäke | 2 |
| Natriumhydroxid (45%ig) | 0,3 |
| Wasser 100% | Ad 100 |

| | |
|---|---|
| pH: 6,5-7,5 | |

| **Beispielrezeptur 2** | **Gew.-%** |
|---|---|
| Paraffinum Liquidum | 18 |
| Cetylpalmitat | 1 |
| Diisostearoyl Polyglyceryl-3 Dimer dilinoleate | 1,2 |
| Isopropylpalmitat | 4,5 |
| Polyglyceryl-4 Diisostearat/Polyhydroxystearat/Sebacat | 1,2 |
| Magnesiumsulfat | 0,6 |
| Phenoxyethanol | 0,4 |
| Decandiol | 0,1 |
| RonaFlair^{®} Flawless | 3 |
| Glycerin | 5 |
| Wasser 100% | Ad 100 |

| **Beispielrezeptur 3** | **Gew.-%** |
|---|---|
| Cetylstearylalkohol | 2,5 |
| Glycerylstearatcitrat | 2,0 |
| Hydrogenated Coco-Glycerides | 1 |
| Dimethicone | 1 |
| Benzethoniumclorid | 0,05 |
| Ethylhexylglycerin | 0,5 |
| Carbomer | 0,3 |
| C12-15 Alkylbenzoat | 2 |
| Caprylic/Capric triglyceride | 2 |
| Dicaprylylether | 3 |
| Ethylhexylcocoat | 2 |
| Natriumhydroxid (45%ig) | 0,1 |
| Benzykalkohol | 0,2 |
| RonaFlair^{®} Flawless | 3 |
| Glycerin | 5 |
| Wasser 100% | Ad 100 |

| | |
|---|---|
| pH: 5.5-7 | |

| **Beispielrezeptur 4** | **Gew.-%** |
|---|---|
| Natriumstearoylglutamat | 0,2 |
| Cetylstearylalkohol | 2 |
| Glyceryl Stearate | 1 |
| Dimethicone / Dimethicone-Crosspolymer | 10 |
| Dimethicone | 4 |
| Caprylic/Capric Triglyceride | 3 |
| Dicaprylylether | 3 |
| Butylmethoxydibenzoylmethan | 1 |
| Octocrylen | 2 |
| Octyl Salicylate | 3 |
| Phenoxyethanol | 0,5 |
| Ubichinon | 0,05 |
| Glycerylcaprylat | 0,5 |
| Xanthan Gummi | 0,1 |
| Carbomer | 0,1 |
| RonaFlair^{®} Flawless | 2 |
| Farbpigmente (CI 77891, CI 77492, CI 77491, CI 77499, CI 77007) | 5 |
| Natriumhydroxid (45%ig) | 0,35 |
| EDTA | 1 |
| Glycerin | 9 |
| Wasser 100% | Ad 100 |

| | |
|---|---|
| pH: 5.5-7 | |

| **Beispielrezeptur 5** | **Gew.-%** |
|---|---|
| Cetylstearylalkohol | 0,9 |
| Octyldodecanol | 2 |
| Dicaprylylether | 2 |
| Octandiol | 0,5 |
| Glycerystearat | 1,5 |
| Coco-Caprylat/Caprat | 2 |
| Acrylates/C10-30 Alkyl Acrylate Crosspolymer | 0,2 |
| Natrium Cetylstearylsulfat | 0,2 |
| Dimethicone/Dimethicone Crosspolymer | 20 |
| Ethanol denat. | 3 |
| Tapiocastärke | 2 |
| Phenoxyethanol | 0,3 |
| RonaFlair^{®} Flawless | 3 |
| Glycerin | 9 |
| Natriumhydroxid (45%ig) | 0,2 |
| Wasser 100% | Ad 100 |

| | |
|---|---|
| pH-Wert: 6.5-7.5 | |

## Patentansprüche

1. Kosmetische oder dermatologische Zubereitungen mit einem Gehalt an sphärischen Silicapartikeln, welche mit Titandioxid und Eisenoxiden beschichtet sind, welche gewählt werden aus der Gruppe der "Soft-Focus-Pigmente",
und
wobei die sphärischen Partikel eine mittlere Korngröße von 4.0 - 8.0 µm und folgende typische Zusammensetzung haben: 74.0 - 85.0 % Siliciumdioxid (Silica), 15.0 - 24.0 % Titandioxid und ≤ 2.0% Eisenoxide.
und mittelkettigen aliphatischen Diolen, welche gewählt werden aus der Gruppe Octandiol, Ethylhexylglycerin und/oder Glycerylcaprylat.

2. Zubereitungen nach Anspruch 1, **dadurch gekennzeichnet, daß** sie 0,01 - 30 Gew.-%, bevorzugt 0,1 - 20 Gew.-%, besonders bevorzugt 0,2 - 10 Gew.-% an einem oder mehreren der erfindungsgemäßen mittelkettigen aliphatischen Diole enthalten, bezogen auf die Gesamtzusammensetzung der Zubereitungen, gewählt aus der Gruppe Octandiol, Ethylhexylglycerin und Glycerylcaprylat

## Claims

1. Cosmetic or dermatological preparations having a content of spherical silica particles coated with titanium dioxide and iron oxides selected from the group of "soft-focus pigments",
and
wherein the spherical particles have a mean particle size of 4.0 - 8.0 µm and the following typical composition: 74.0 - 85.0% silicon dioxide (silica), 15.0 - 24.0% titanium dioxide and ≤ 2.0% iron oxides
and medium-chain aliphatic diols selected from the group of octanediol, ethylhexylglycerin and/or glyceryl caprylate.

2. Preparations according to Claim 1, **characterized in that** said preparations comprise 0.01 - 30% by weight, preferably 0.1 - 20% by weight, particularly preferably 0.2 - 10% by weight, based on the total composition of the preparations, of one or more medium-chain aliphatic diols according to the invention selected from the group of octanediol, ethylhexylglycerin and glyceryl caprylate.

## Revendications

1. Préparations cosmétiques ou dermatologiques comportant une teneur en particules sphériques de silice qui sont revêtues par du dioxyde de titane et des oxydes de fer, qui sont choisies dans le groupe des « pigments soft focus »,
et
les particules sphériques ayant une taille moyenne de grain de 4,0 à 8,0 µm et ayant une composition typique suivante : 74,0 à 85,0 % de dioxyde de silicium (silice), 150 à 24,0 % de dioxyde de titane et ≤ 2,0 % d'oxydes de fer,
et des diols aliphatiques à longueur de chaîne moyenne, qui sont choisis dans le groupe de l'octanediol, l'éthylhexylglycérine et le caprylate de glycéryle.

2. Préparations selon la revendication 1, **caractérisées en ce qu'**elles contiennent 0,01 à 30 % en poids, préférablement 0,1 à 20 % en poids, particulièrement préférablement 0,2 à 10 % en poids d'un ou plusieurs diols aliphatiques à longueur de chaîne moyenne selon l'invention, par rapport à la composition totale des préparations, choisis dans le groupe de l'octanediol, l'éthylhexylglycérine et le caprylate de glycéryle.
